(19) Europäisches Patentamt

European Patent Office

Office européen des brevets

(11) **EP 1 013 269 A1**

(12) **EUROPEAN PATENT APPLICATION**
published in accordance with Art. 158(3) EPC

(43) Date of publication:
**28.06.2000 Bulletin 2000/26**

(51) Int. Cl.⁷: **A61K 9/127**, A61K 9/107,
A61K 9/14, A61M 13/00,
A61M 15/00

(21) Application number: **98921874.8**

(22) Date of filing: **29.05.1998**

(86) International application number:
**PCT/JP98/02374**

(87) International publication number:
**WO 98/55104 (10.12.1998 Gazette 1998/49)**

(84) Designated Contracting States:
**AT BE CH DE DK ES FI FR GB GR IE IT LI LU MC
NL PT SE**

(30) Priority: **06.06.1997 JP 14834697**

(71) Applicant: **SHIONOGI & CO., LTD.
Osaka-shi, Osaka 541-0045 (JP)**

(72) Inventors:
• **NAGATA, Shunji
Ashiya-shi, Hyogo 659-0032 (JP)**

• **KANAOKA, Eri
Osaka-shi Osaka 533-0006 (JP)**

(74) Representative:
**Baverstock, Michael George Douglas
BOULT WADE TENNANT,
Verulam Gardens
70 Gray's Inn Road
London WC1X 8BT (GB)**

(54) **IMPROVEMENT IN MEDICAMENT ADMINISTRATION SYSTEM**

(57) A drug preparation to be administered by a medicament administration device, which can maintain high stability of a physiologically active substance. In preparing pharmaceutical preparations for mucosal application, particularly a pharmaceutical preparation to be inhaled by utilizing a jet nebulizer, an ultrasonic nebulizer, a constant rate sprayer, or a powder inhaler, the adoption of the step of contact with liposome or lipid microspheres in an aqueous medium enables a physiologically active substance to be highly stabilized.

EP 1 013 269 A1

Printed by Xerox (UK) Business Services
2.16.7 (HRS)/3.6

## Description

### Technical Field

The present invention relates to a kit for preparing a pharmaceutical formulation and the like, which comprises a preparation of a biologically active substance and a preparation containing a liposome or the like, said preparations being used for production of a pharmaceutical formulation to be administered by a medicament administration device that disperses the biologically active substance in the air, said production being conducted by contacting the biologically active substance with a liposome or the like in an aqueous medium; a method for stabilizing a biologically active substance by contacting the biological active substance with a liposome or the like in an aqueous medium in the process for the production of a pharmaceutical formulation to be administered by a medicament administration device that disperses the biologically active substance in the air; and the pharmaceutical formulation and the like.

### Background Art

Many attempts have recently been made for utilizing biologically active substances such as peptides, proteins, nucleic acids, etc. as medicaments. Such biologically active substances used for this purpose must have potent pharmaceutical activity and high stability. It is known that biologically active substances administered by the oral route are either decomposed by digestive enzymes or poorly absorbed. However, administration of the substances by injection gives pain to patients. Accordingly, administration via mucous membrane is preferred because it minimizes a patients' physical pain and contributes to the increase of pharmaceutical activity and stability of the biologically active substances.

However, biologically active substances such as peptides, proteins, nucleic acids, etc., more specifically, cytokines, proteases, bone-related peptides, are liable to decompose or lose their biological activities when administered via mucous membrane in the form of a pharmaceutical formulation, especially by the use of a medicament administration device such as a nebulizer, and therefore, sufficient therapeutic effect cannot be obtained.

In this situation, various devices and ideas have been proposed so that biologically active substances such as peptides, proteins, and nucleic acids, etc. may fully display their activities. *Inter alia*, a formulation prepared by contacting biologically active substances with a liposome is known (Japanese Patent Publication (not examined) No. 82839/1992; Japanese Patent Publication (not examined) No. 99897/1996). Japanese Patent Publication (not examined) No. 82839/1992 discloses an attempt for prolongation of the active duration of peptidic or proteinic biologically active substances by the use of charged lipids, to the membrane surface to which the substances bind. Japanese Patent Publication (not examined) No.99897/1996 discloses that IL-2-adsorbed lipid spheres preparation may be conducted by contacting IL-2 with liposome or the like in an aqueous medium. However, it only discloses that there is no relation between the type of the preparation and electric charge, and that the preservation rate of IL-2 is increased. Thus, the above-noted publications neither disclose nor suggest that biologically active substances such as peptides, proteins, nucleic acids, and the like, which hitherto have not been developed for a medicament without difficulties mainly due to their characteristic-liability to decomposition or loss of their biologically activities, can be stabilized by formulating the substances in the form of a formulation to be administered via a mucous membrane.

### Disclosure of Invention

The present invention solves the problems mentioned above, and the object of the invention is to provide a kit capable of readily preparing a pharmaceutical formulation having a high stability with respect to a biologically active substance which is to be administered by a medicament administration device that disperses the substance in the air, such as, in particular, a jet nebulizer, an ultrasonic nebulizer, a metered dose inhaler, a dry powder inhaler, etc., said device causing a decrease of the stability of the substance; to provide a method for the production thereof; and to provide the pharmaceutical formulation.

The inventors of the present invention have found, after extended studies, that when preparing a pharmaceutical formulation which is inhaled using a jet nebulizer, an ultrasonic nebulizer, etc., high stability of a biologically active substance could be attained by adopting the step of contacting the substance with liposomes or microspheres.

Thus, the present invention provides a kit for preparing a pharmaceutical formulation for a human or animal, which comprises a preparation of a biologically active substance and a preparation containing liposomes or microspheres, which preparations are used for production of the pharmaceutical formulation to be administered by a medicament administration device that disperses the biologically active substance in the air, said production being conducted by contacting the biologically active substance with liposomes or microspheres in an aqueous medium.

According to the preferred embodiment of the kit, the medicament administration device for dispersing the biologically active substance in the air is a jet nebulizer, an ultrasonic nebulizer, a metered dose inhaler, or a dry powder inhaler.

The pharmaceutical formulation for a human or animal is preferably one which is administered via a mucous membrane.

The biologically active substance is preferably one which loses its stability when dispersed in the air. Examples of such substances are peptides, proteins, or nucleic acids. Preferred peptides are cytokines and bone related peptides, and preferred proteins are proteinases, antibiotic proteins, and anti-tumor proteins. Nucleic acids are preferably DNA, RNA, or their antisenses. Among cytokines, interferons are preferred, and interferon $\gamma$ to be used for curative and prophylactic treatment of virus infections or pulmonary diseases is more preferred.

A preparation of a biologically active substance and/or a preparation containing liposomes or microspheres is or are preferably in the form of an aqueous dispersion. However, a kit including a container containing an aqueous medium is also preferable. Further, a preparation of a biologically active substance and/or a preparation containing liposomes or microspheres is or are also preferably in the form of a lyophilized preparation.

Other objects of the present invention are as follows:

The present invention provides a method for preparing a pharmaceutical formulation for a human or animal to be administered by a medicament administration device that disperses a biologically active substance in the air, which comprises the step of contacting the biologically active substance with liposomes or microspheres in an aqueous medium.

The present invention also provides a method for stabilizing a biologically active substance by contacting the biologically active substance with liposomes or microspheres in the process for the production of a pharmaceutical formulation for a human or animal to be administered by a medicament administration device that disperses the biologically active substance in the air.

The present invention further provides a pharmaceutical formulation for a human or animal to be administered by a medicament administration device that disperses a biologically active substance in the air, said formulation having been produced by contacting the biologically active substance with liposomes or microspheres in an aqueous medium.

The invention additionally provides a method for curative or prophylactic treatment of diseases of humans or animals, which comprises administering to the human or the animal a pharmaceutical formulation for a human or animal to be administered by a medicament administration device that disperses a biologically active substance in the air, said formulation having been produced via a step of contacting the biologically active substance with liposomes or microspheres in an aqueous medium.

The present invention further provides use of liposomes or microspheres for the production of a pharmaceutical formulation for a human or animal to be administered by a medicament administration device that disperses a biologically active substance in the air, said formulation having been produced via a step of contacting the biologically active substance with liposomes or microspheres in an aqueous medium.

The present invention furthermore provides a metered dose inhaler or a dry powder inhaler, characterized in that it contains a pharmaceutical formulation for a human or animal to be administered by a medicament administration device that disperses a biologically active substance in the air, said formulation having been produced via a step of contacting the biologically active substance with liposomes or microspheres in an aqueous medium.

The preferred embodiments for the pharmaceutical formulation for a human or animal are also applicable to all of the six inventions mentioned above.

The kit of the present invention is in general provided in the form of a container such as a bottle, containing a preparation of a biologically active substance or a preparation containing liposomes or microspheres. The pharmaceutical formulation for a human or animal of the invention, which was prepared by the method for production thereof or the method for stabilizing a biologically active substance of the invention, is used by employing a medicament administration device that disperses the biologically active substance in the air. The pharmaceutical formulation for a human means a composition used for curative or prophylactic treatment of a human, which contains a biologically active substance, while the pharmaceutical formulation for an animal means a composition used for curative or prophylactic treatment of an animal, which contains a biologically active substance. The preparation of a biologically active substance or the preparation containing liposomes or microspheres means a liquid or powder composition containing the biologically active substance or the liposomes or microspheres. When both or either of the preparation of a biologically active substance and the preparation containing liposomes or microspheres is or are a dried preparation or preparations and the other is an aqueous solution or suspension, the kit of the present invention may be prepared by mixing the preparations when used, while adding an aqueous medium thereto if necessary. In this instance, the kit of the present invention may contain a container such as a bottle which contains an aqueous medium having its pH properly adjusted. The kit of the present invention includes one which is produced by preparing an aqueous medium containing liposomes or microspheres and a biologically active substance, and subsequently lyophilizing the medium.

The medicament administration device that disperses a biologically active substance used in the present invention means a device for delivering and administrating the biologically active substance, for instance, in the form of an aerosol, to patients by applying physical energy such as pressure to the substance so that it may reach a mucous membrane of the airway or alveolus. Such a device includes a nebulizer, a metered dose inhaler, a dry powder inhaler, etc. These devices are explained in detail below, generally according to the teaching in Pharmacia, 33(4), p370 - 381, 1997.

The nebulizer may be essentially classified as an ultrasonic nebulizer and a jet nebulizer. The ultrasonic nebulizer is a device which generates an aerosol by means of molecular movement caused by ultrasonic vibration. The jet nebulizer is a device which generates an aerosol by means of compressed gas released from a compressor or a gas cylinder. These nebulizers often disperse in the air a solution of medicament which is accommodated in a bottle, ampoule, etc., after the solution is introduced into a medicament chamber (a spray chamber) when used. Such nebulizers are useful for infants, the aged, and patients who are under control of a respirator due to lack of spontaneous respiration. As much as 50% of the medicament solution sometimes remains in the spray chamber after use. Jet nebulizers may be divided into two types, one of which applies air pressure to a solution to be sprayed, and the other applies vapor pressure to a solution to be sprayed.

The metered dose inhaler (MDI), for instance, a pressurized metered dose inhaler (pMDI), is a device in which an aerosol is generated when a biologically active substance dissolved or suspended in a liquefied gas called a propellant as a nebula, which is stored in a pressure resistant airtight container, is released from the container(see Drug Delivery to the Respiratory Tract, Ellis Horwood, edited by D. Ganderton and T. Jones, p87 - 118, 1987). The metered dose inhaler is portable and ready for instant use, although it requires proficiency in inhalation technique to harmonize the release with the inhalation. For this purpose, an inhalation aid called a spacer has been developed.

An MDI may be produced by a cooling method or a compression method. In the former, a biologically active substance without chlorofluorocarbon is mixed and suspended with the chlorofluorocarbon, and the mixture is cooled and admixed with pre-cooled chlorofluorocarbon, and the admixture is divided into small portions. A separately molded quantitative valve is attached thereto. In the latter, a biologically active substance is placed in a container. After a quantitative valve is attached thereto, chloro-fluorocarbon mixture is added through a valve stem under pressure. Alternatively, a biologically active substance suspended in chloro-fluorocarbon is charged under pressure (see Clark A.R., Aerosol Science Technology, 22, 374, 1995; Pharm. Tech. Jpn., 2(10), 1056, 1986). A typical commercial product is Probentil HFA (3M Pharmaceuticals) and so on.

A dry powder inhaler (DPI) is a device which generates an aerosol by making a hole in a capsule or a disc in which a biologically active substance is accommodated, whereby the substance is dispersed when inhaled. This inhaler is classified into three types, a capsule type, a blister type(unit dose), and a reservoir type. A DPI requires, unlike a pMDI, a patients' efforts for inhalation of a biologically active substance. Typical commercial products are Spinhaler(Fisons), Diskhaler (Allen & Hanburys), Turbuhaler (Astra Draco) (see Naoki FUJIMURA, Kokyu (respiration), 15, 502, 1996), Rotahaler (Allen & Hanburys), Cyclohaler (Pharbita), Inhalater-M (Boehringer Ingelheim), Aquhaler (Allen & Hanburys), Easyhaler (Orion), and the like. When a dry powder inhaler is used, it is preferable that the pharmaceutical formulation for a human or an animal is a lyophilized product.

A preferred embodiment of "administration via mucous membrane" is, among administration embodiments wherein a biologically active substance is scheduled to exert its activity on the mucous membrane or to exert its activity after having been absorbed via a mucous membrane, an embodiment wherein the biologically active substance loses its stability, that is, decomposes or loses its activity, due to physico-chemical factors such as the physical energy of ultrasonic waves, energy generated by the flow current on the interface between a drop of water and outside air, and the like, when a medicament administration device such as a jet nebulizer, an ultrasonic nebulizer , a dry powder inhaler, or a metered dose inhaler is used. However, in other administration embodiments wherein the stability of the biologically active substance does not decrease to a great extent, it appears that the present invention can attain stabilization of the substance. Mucous membranes in the body which absorb a physiologically inactive substance are, for instance, the nasal mucous membrane, airway mucous membrane, the bronchial mucous membrane, the pulmonary mucous membrane and the like.

The "step of contacting a biologically active substance to be administered by a medicament administration device with liposomes or microspheres in an aqueous medium" may be carried out in a vessel called a medicament chamber in a jet nebulizer, an ultrasonic nebulizer, a metered dose inhaler, or a dry powder inhaler, or outside the vessel (after the contact, the mixture is introduced into the medicament chamber). The step may be carried out by means of an inhalation system and device which allows a gradual or arbitrary contact of a biologically active substance to be administered by a medicament administration device with liposomes or microspheres. It should be noted that according to the present invention, the phrase "the step of contacting a biologically active substance with lipid microspheres and the like in an aqueous medium" does not include an embodiment in which the biologically active substance is incorporated in a liposome.

A "biologically active substance to be administered by a medicament administration device" used in the present invention includes, for instance, fibrinolytic agents, anticoagulants, hematopoietics, antibiotics, treatment agents for infectious diseases, anti-dementia agents, antiviral agents, antitumor agents, antipyretics, analgesics, anti-inflammatory agents, antiulcer agents, antiallergic agents, psychotropic agents, cardiacs, anti-arrhythmic agents, vasodilators, anti-hypertensive agents, anti-diabetic agents, cholesterol lowering agents, osteoporosis treating agents, hormones, vaccines, activated oxygen inhibitors, when described according to therapeutic effects. The biologically active substances include, for example, cytokines (e.g. interferon γ and interleukin 2), bone-related peptides (e.g. calcitonin, bone morphogenetic protein, growth hormone), and the like, when described according to the properties of the substances.

Preferred substances on which the effect of the present invention is obtained are, among other biologically active substances mentioned above, those which lose their stability through decomposition or loss of activity, due to physico-chemical factors such as physical energy of ultrasonic waves, energy generated by a flow current on the interface between a drop of water and outside air, and the like, when a medicament administration device such as a jet nebulizer, an ultrasonic nebulizer, a dry powder inhaler, or a metered dose inhaler is used. However, biologically active substances, stabilities of which do not decrease significantly when dispersed in the air, can also obtain the advantage of the stabilizing method of the present invention. Accordingly, the biologically active substances to be used in the present invention are not limited to those listed below, which are exemplified as being preferred, as far as they exert their activities on mucous membrane or they are absorbed via a mucous membrane. That is, all bioactive substances, when administered in the form of a pharmaceutical formulation to be administered via a mucous membrane, can be improved in terms of their stabilities by the method of the present invention.

As stated above, the biologically active substances are preferably those which lose their stabilities when dispersed in the air, such as peptides, proteins, nucleic acids and the like.

Specific examples of the peptides or proteins are angiotensin, insulin, insulin-like growth factors, interferon α, interferon β, interferon γ, interleukin 1, interleukin 2, interleukin 2 receptor regulatory factor, interleukin 3, urokinase, erythropoietin, enkephalin, peptides having endothelin antagonistic activity, endorphin α, endorphin β, endorphin γ, lysozyme chloride, luteinizing hormone, oxytocine, gastrin, kalikrein, calcitonin, tumor destroying factor, thymus humoral factor, kyotorphin, class-selective suppressor factor, gramicidin, glucagon, blood coagulation factors (Factor VIII, Factor IX), thymus factor in blood, thyroid-stimulating hormone, thyroid hormone releasing hormone, bone morphogenetic grotein, gonadotropin, gonadoliberin, colistin, corticoliberin, cholecystokinin, colony-stimulating factor, cytotoxic T lymphocyte differentiation-inducing factor, cytotoxic T lymphocyte inducer, cell proliferation factor, thymosin, thymostimulin, thymopoietin, Substance P, tumor necrosis factor (TNF),epidermal growth factor, T cell growth factor, neurotrophic factor, nerve growth factor, scotophobin, growth hormone, secretin, zenobsin, caerulein, somatostatin, somatomedin, somatoliberin, dynorphin, tuftsin, thyroliberin, tissue plasminogen activator, T cell substitutes, thrombopoietin, neurotensin, paracrine, pancreozymin, bacitracin, vasopressin, B lymphocyte growth factor, B lymphocyte differentiation factor, B lymphocyte maturation factor, human cilium gonadotropin, human placenta lactogen, parathyroid-stimulating hormone, adrenocorticotropic hormone, bradykinin, prolactin, bombesin, polymyxin B, macro phage-activating factor, macrophage-migration inhibitory factor, melanocyte-stimulating hormone, motilin, ubiquitin, repressible cell-inducing factor, inhibitory B factor, folicle-stimulating factor, lymphocyte-activating factor, renin, proteolytic enzymes (e.g. expectorants, cathepsins), antibiotic proteins (e.g. acacin), antitumor proteins (e.g. L-asparaginase, neocarzinostatin), superoxide dismutase, and the like.

Nucleic acids include DNA, RNA, and their antisenses.

Biologically active substances other than peptides, proteins, and nucleic acids are lipopolysaccharides, fluorouracil, doxorubicine, mitomycin C, platinum complex, cyclophosphamide, actinomycin, polyoxin, and the like.

Biologically active substances include pharmacologically acceptable salts thereof. For instance, salts of a biologically active substance having a basic group such as an amino group, with an inorganic acid (e.g. hydrochloric acid, sulfuric acid, nitric acid, etc.) or an organic acid (e.g. carbonic acid, succinic acid, etc.) and salts of a biologically active substance having an acidic group such as a carboxyl group, with an inorganic base (e.g. alkali metals such as sodium or potassium), or an organic base (e.g. triethylamine, arginine, etc.) are included in the biologically active substances.

Liposome is a closed vesicle comprising double layer phospholipid membranes, which can retain various biologically active substances in an internal aqueous layer or phospholipid double layer. Accordingly, extensive studies on liposome as a drug carrier have long been conducted. The preferred particle size of the liposome is about 20 nm - 3μm. Lipid microspheres, also called lipid emulsions, are small lipid particles obtained by, for example, suspending plant oil such as soybean oil in water in the presence of phospholipid such as lecithin. The average particle size of lipid microspheres is preferably about 100 nm - 300 nm. It should be noted that other aqueous media into which similar lipids such as phospholipids, synthetic surfactants etc. are dispersed, that is, emulsions, mixed micelles, microcapsules, and microspheres are different from the liposome or lipid microspheres according to the invention (Fragrance Journal, Vol. 15, No. 6, p68 - 76, 1987).

Major components of phospholipids constituting liposomes or microspheres used in the present invention are not restrictive and may be neutral phospholipids or charged lipids as described in the afore-mentioned Japanese Patent Publication (Kokai) No. 9989711996. However, a suitable combination of a medicament and liposome (kind and ratio of components of membrane) will exist for establishing better stability of the biologically active substance used. Neutral phospholipids are not limitative as long as they are phospholipids having no charge in their neutral region. For instance, synthetic or natural phosphatidyl choline, sphingomyelin, or a mixture thereof may be used. Natural phosphatidyl cholines may include various lecithins, which may be the unsaturated or saturated type (hydrogenated type), and include distearoyl phosphatidyl choline, dipalmitoyl phosphatidyl choline, dimyristoyl phosphatidyl choline, dibehenoyl phosphatidyl choline, dilignoceroyl phosphatidyl choline, sphingomyelin, egg yolk lecithin, soybean lecithin, hydrogenated egg yolk lecithin, hydrogenated soybean lecithin, and the like. One or more lipids selected from the above may be used.

Phospholipids for liposome or lipid microspheres used in the production of the pharmaceutical formulation of the present invention may be charged phospholipids. Positively-charged phospholipids may be used without limitation, as long as they are not toxic. These are, for instance, basic phospholipids such as stearyl amine, oleyl amine, etc., and basic amino acid surfactants such as $N^a$-acyl-L-arginine. Negatively-charged phospholipids may also be used without limitation, as long as they are not toxic. These are, for instance, acidic phospholipids such as phosphatidyl serine, phosphatidyl glycerol, dimyristoyl phosphatidyl glycerol, dipalmitoyl phosphatidyl glycerol, distearoyl phosphatidyl glycerol, cardiolipin, phosphatidyl inositol, phosphatidic acid, etc., acidic lipids such as gangliosides, dicetylphospholic acid, etc., acidic amino acid surfactants such as N-acyl-L-glutamic acid, and fatty acids such as oleic acid, stearic acid, etc.

Major lipids, which are used together with the phospholipids mentioned above, for constructing microspheres used for the production of the pharmaceutical formulation of the invention, are plant oils (e.g. soybean oil, sesame oil, safflower oil, etc.), synthetic or semi-synthetic middle chain fatty acid triglycerides (e.g. migliol, ODO (registered trade name), etc.).

To the lipid components of the liposomes or microspheres, phosphatidyl ethanolamine, sterols such as cholesterol as a membrane stabilizer, and $\alpha$-tocopherol as an antioxidant may be added, in addition to the above-mentioned various phospholipids and electrically charged lipids. The amounts of such additives to be added are as follows: When the total amount of the lipids is defined as 100 parts by weight, phosphatidyl ethanolamine is 0.01 - 30 parts by weight, a sterol used as a stabilizer is 0.01 - 100 parts by weight, preferably 20 - 55 parts by weight, and $\alpha$-tocopherol used as an antioxidant is 0.01 - 20 parts by weight, preferable about one part by weight.

The method for the production of liposomes or microspheres of the present invention is not limited and may be carried out in different ways as in the aforementioned Japanese Patent Publication (Kokai) No. 99897/1996. For instance, liposome may be produced by the ultrasonic method, the vortex method, the detergent method, the ethanol injection method, the ether injection method, the French press method, etc, the reverse phase evaporation method, and the high pressure emulsifying method. The structures of the liposome prepared by these methods may be multilamellar vesicle (MLV), small unilamellar vesicle (SUV), and reverse-phase evaporation vesicle (REV). On the other hand, the production of lipid microspheres is conducted, unlike the production of liposome, by the use of a plant oil such as soybean oil, sesame oil, and the like, or synthetic or semi-synthetic middle chain fatty acid triglycerides in addition to phospholipids. The method of the production of the microspheres is not limited, and the production may be carried out, for example, by adding 2 - 20% plant oil or middle chain fatty acid triglyceride and 12% (with respect to the plant oil or triglyceride) of phospholipid to water and subjecting the resultant mixture to a high pressure emulsifier to obtain lipid microspheres. The liposome and lipid microspheres may be chemically or physically coated with hydrophilic polymers such as polyethylene glycol, pullulan, mannan, polyamino acid and the like.

Preferred liposomes or microspheres used in the present invention are those which do not internally contain a biologically active substance, that is, empty liposome and the like. However, the liposomes or microspheres may contain, distribute, or adsorb other biologically active substances before contact with a biologically active substance in the present invention in an aqueous medium. The other biologically active substances are not limited.

The aqueous medium used in the present invention may contain various stabilizers depending on the kind of particular biologically active substance used. As the stabilizer, saccharides such as monosaccharides (e.g. glucose, galactose, etc.), disaccharides (e.g. maltose, sucrose, etc.), and polysaccharides (e.g. dextran), polyols such as glycerol, mannitol, sorbitol, xylitol, and the like, water-soluble polymers such as gelatin, hydroxyethyl starch, surfactants, and the like may be mentioned.

The pharmaceutical formulation of the present invention may contain known absorption enhancing agents depending on the nature of particular biologically active substance to be administered using a medicament administration device. In addition, additives for different purposes, such as surfactants, stabilizing agents, buffers, isotonic agents, antiseptics may be added to the formulation.

Dosage of the biologically active substance to be administered by a medicament administration device varies greatly depending on the nature of the substance, duration of the treatment, and other factors. The ratio of the amount of the biologically active substance to be administered with a medicament administration device and the amount of liposome or microspheres, which are contacted, also varies greatly depending on the nature of particular biologically active substance and other factors. For instance, where the biologically active substance is interferon, 40 nmol thereof is used with 1 - 1000 $\mu$mole of phospholipid.

The terms "residual rate" and "spraying rate" used in the working examples described hereinafter will be explained below.

The residual rate is calculated according the following equation.

$$\text{Residual rate} = \frac{\text{Amount of biologically active substance remaining in medicament chamber of device}}{\text{Total amount of biologically active substance initially charged}} \times 100$$

(Equation 1)

The greater the residual rate, the higher the stability of the biologically active substance in the medicament chamber, in which the substance remains undecomposed or without losing its biologically activity.

The spraying rate is expressed by the following equation.

$$\text{Spraying rate} = \frac{\text{Amount of biologically active substance remaining in capturing solution}}{\text{Total amount of biologically active substance initially charged}} \times 100 \quad \text{(Equation 2)}$$

The greater the spraying rate, the higher the stability of the biologically active substance sprayed, without decomposition and losing its physiological activity.

**Best mode of the present invention**

**EXAMPLES**

The invention will be explained in more detail with Reference Examples, Working Examples, and Experiments. Reference Examples are directed to the production of liposome and lipid microspheres used in the present invention, and Working Examples are directed to the production of the pharmaceutical formulation of the invention to be administered via the mucous membrane. The Experiments show that the pharmaceutical formulation of the invention to be administered via the mucous membrane is kept stable in a medicament administration device such as a jet nebulizer, an ultrasonic nebulizer, and the like.

**Reference Example 1**

Production of liposome (HSPC/DSPG = 10/1)

Five ml of a tert-butanol solution (containing 1 ml of distilled water) containing a mixture of 100 mg of hydrogenated soybean lecithin (HSPC) and 10 mg of distearoylphosphatidyl glycerol (DSPG) was lyophilized. To the resultant lipid mixture was added and dispersed 10 ml of a phosphate buffer (10% maltose). The dispersion was subjected to a Nanomizer-LA-10H (Sayama trade name) ten times under a treatment pressure of 2.4kg/cm$^2$, while warming at 60°C, to give liposome (HSPC/DSPG=10/1).

**Reference Example 2**

Production of liposome (DSPC/DPPG = 10/1)

Reference Example 1 was repeated except that a mixture of 100 mg of distearoylphosphatidyl choline (DSPC) and 10 mg of dipalmitoylphosphatidyl glycerol (DPPG) was used in place of a mixture of 100 mg of hydrogenated soybean lecithin (HSPC) and 10 mg of distearoylphosphatidyl glycerol (DSPG).

**Reference Example 3**

Production of liposome (EggPC/DSPG = 10/1)

Reference Example 1 was repeated except that a mixture of 100 mg of egg yolk lecithin (EggPC) and 10 mg of distearoylphosphatidyl glycerol (DSPG) was used in place of a mixture of 100 mg of hydrogenated soybean lecithin (HSPC) and 10 mg of distearoylphosphatidyl glycerol (DSPG), and the mixture was kept at 4°C rather than 60°C.

**Reference Example 4**

Production of liposome (DPPC/CH/DPPG = 54/40/6)

Reference Example 1 was repeated except that a mixture of 54 mg of dipalmytoylphosphatidyl choline (DPPC), 20 mg of cholesterol (CH), and 6mg of dipalmytoylphosphatidyl glycerol (DPPG) was used in place of a mixture of 100 mg of hydrogenated soybean lecithin (HSPC) and 10 mg of distearoylphosphatidyl glycerol(DSPG).

### Reference Example 5

Production of liposome (EggPC)

Reference Example 1 was repeated except that 100 mg of egg yolk lecithin (EggPC) was used in place of a mixture of 100 mg of hydrogenated soybean lecithin (HSPC) and 10 mg of distearoylphosphatidyl glycerol (DSPG), and the treatment was conducted at 4°C rather than 60°C.

### Reference Example 6

Production of liposome (DPPC/CH/SA = 52/40/8)

Reference Example 1 was repeated except that a mixture of 52 mg of dipalmytoylphosphatidyl choline (DPPC), 20 mg of cholesterol (CH), and 8mg of stearyl amine (SA) was used in place of a mixture of 100 mg of hydrogenated soybean lecithin (HSPC) and 10 mg of distearoylphosphatidyl glycerol (DSPG).

### Reference Example 7

Production of lipid microspheres

A crude emulsion was obtained by mixing 216 mg of DSPC, 24 mg of DSPG, 500 mg of glycerol, and 20 ml of distilled water for injection, dispersing the mixture using a homogenizer (Polytron, registered trade name), and subsequently treating the mixture with the homogenizer after addition of 2 gram of soybean oil. The resulting mixture was emulsified with a Nanomizer-LA-10H (Sayama trade name) to give lipid microspheres having an average particle size of about 200 nm.

### Example 1

Twenty ml of a solution containing recombinant interferon $\gamma$ (IFN-$\gamma$; 3 million units), said solution containing human serum albumin and L-cysteine hydrochloride as stabilizers, was introduced into the medicament chamber of an ultrasonic nebulizer (Omuron: NE-U12). Addition of a liposome suspension (125 $\mu$mole as phospholipid) thereto gave a pharmaceutical formulation for a human or animal.

### Example 2

Five ml of a solution containing recombinant interferon $\gamma$ (IFN-$\gamma$; 3 million units), said solution containing human serum albumin and L-cysteine hydrochloride as stabilizers, was introduced into a medicament chamber of a jet nebulizer (Nippon Shoji; Nissho style). Addition of a liposome suspension (125 $\mu$mole as phospholipid) thereto gave a pharmaceutical formulation for a human or an animal.

### Example 3

Five ml of a solution containing recombinant interferon $\gamma$ (IFN-$\gamma$; 3 million units), said solution containing human serum albumin and L-cysteine hydrochloride as stabilizers, was introduced into the medicament chamber of a metered dose inhaler. Addition of a liposome suspension (125 $\mu$mole as phospholipid) thereto gave a pharmaceutical formulation for a human or animal.

### Example 4

Five ml of a solution containing 2.5 $\mu$mole of urokinase was introduced into the medicament chamber of a jet nebulizer. Addition of a liposome suspension (125 $\mu$mole as phospholipid) thereto gave a pharmaceutical formulation for a human or an animal.

### Example 5

Twenty ml of a solution containing 2.5 μmole of urokinase was introduced into the medicament chamber of an ultrasonic nebulizer. Addition of a liposome suspension (125 μmole as phospholipid) thereto gave a pharmaceutical formulation for a human or animal.

### Example 6

Five ml of a solution containing 70 nmole (one million unit) of recombinant interleukin 2 (IL-2) was introduced into the medicament chamber of a jet nebulizer. Addition of a liposome suspension (125 μmole as phospholipid) thereto gave a pharmaceutical formulation for a human or animal.

### Example 7

Twenty ml of a solution containing 2.5 μmole of L-asparaginase was introduced into the medicament chamber of an ultrasonic nebulizer. Addition of a liposome suspension (125 μmole as phospholipid) thereto gave a pharmaceutical formulation for a human or an animal.

### Example 8

Five ml of a solution containing 2.5 μmole of superoxide dismutase was introduced into the medicament chamber of a jet nebulizer. Addition of a liposome suspension (125 μmole as phospholipid) thereto gave a pharmaceutical formulation for a human or animal.

### Example 9

Twenty ml of a solution containing 2.5 μmole of cathepsin was introduced into the medicament chamber of an ultrasonic nebulizer. Addition of a liposome suspension (125 μmole as phospholipid) thereto gave a pharmaceutical formulation for a human or animal.

### Example 10

Five ml of a solution containing 2.5 μmole of neocarzinostatin was introduced into the medicament chamber of a jet nebulizer. Addition of a liposome suspension (125 μmole as phospholipid) thereto gave a pharmaceutical formulation for a human or animal.

### Example 11

Twenty ml of a physiological phosphate saline (pH 7.4) containing 0.4 μmole of calcitonin was introduced into the medicament chamber of an ultrasonic nebulizer. Addition of a liposome suspension (125 μmole as phospholipid) thereto gave a pharmaceutical formulation for a human or animal.

### Example 12

Twenty ml of a solution containing 3.5 μmole of doxorubicin hydrochloride was introduced into the medicament chamber of an ultrasonic nebulizer. Addition of a liposome suspension (125 μmole as phospholipid) thereto gave a pharmaceutical formulation for a man or animal.

### Example 13

Twenty ml of a solution containing 25 μmole of calcium(+)-(Z)-7-[(1R, 2S, 3S, 4S)-benzensulfonamidobicyclo[2.2.1]hept-2-yl]-5-heptanoate dihydrate was introduced into the medicament chamber of an ultrasonic nebulizer. Addition of a liposome suspension (125 μmole as phospholipid) thereto gave a pharmaceutical formulation for a human or animal.

### Example 14

Five ml of a solution containing 25 μmole of doxorubicin was introduced into the medicament chamber of a jet nebulizer. Addition of a liposome suspension (125 μmole as phospholipid) thereto gave a pharmaceutical formulation for a human or animal.

**Example 15**

Twenty ml of a solution containing 2.5 μmole of interleukin 2 (IL-2) was introduced into the medicament chamber of an ultrasonic nebulizer. Addition of a liposome suspension (125 μmole as phospholipid) thereto gave a pharmaceutical formulation for a human or animal.

**Example 16**

Five ml of a solution containing 2.5 μmole of L-asparaginase was introduced into the medicament chamber of a jet nebulizer. Addition of a liposome suspension (125 μmole as phospholipid) thereto gave a pharmaceutical formulation for a human or animal.

**Example 17**

Twenty ml of a physiological phosphate saline (pH 7.4) containing 0.02 μmole (12000 unit) of superoxide dismutase was introduced into the medicament chamber of an ultrasonic nebulizer. Addition of a liposome suspension (125 μmole as phospholipid) thereto gave a pharmaceutical formulation for a human or animal.

**Example 18**

Five ml of a solution containing 2.5 μmole of cathepsin was introduced into the medicament chamber of a jet nebulizer. Addition of a liposome suspension (125 μmole as phospholipid) thereto gave a pharmaceutical formulation for a human or animal.

**Example 19**

Twenty ml of a solution containing 2.5 μmole of neocarzinostatin was introduced into the medicament chamber of an ultrasonic nebulizer. Addition of a liposome suspension (125 μmole as phospholipid) thereto gave a pharmaceutical formulation for a human or animal.

**Example 20**

Five ml of a solution containing 2.5 μmole of calcitonins was introduced into a medicament chamber of a jet nebulizer. Addition of a liposome suspension (125 μmole as phospholipid) thereto gave a pharmaceutical formulation for a human or animal.

**Example 21**

Five ml of a solution containing 25 μmole of calcium (+)-(Z)-7-[(1R, 2S, 3S, 4S)-benzensulfonamidobicyclo[2.2. 1]hept-2-yl]-5-heptanoate dihydrate was introduced into the medicament chamber of a jet nebulizer. Addition of a liposome suspension (125 μmole as phospholipid) thereto gave a pharmaceutical formulation for a human or animal.

**Example 22**

Five ml of a solution containing recombinant interferon γ (IFN-γ; 3 million units), said solution containing human serum albumin and L-cysteine hydrochloride as stabilizers, was introduced into the medicament chamber of a jet nebulizer. Addition of 120 μl of microspheres obtained in Reference Example 5 thereto gave a pharmaceutical formulation for a human or animal.

**Example 23**

A lyophilized product containing recombinant interferon γ (IFN-γ; 3 million units), said product containing human serum albumin and L-cysteine hydrochloride as stabilizer, was placed into a bottle. Six ml of a liposome suspension (125 μmole as phospholipid) was placed into another bottle. The combination of the two bottles makes a kit.

## Example 24

Twenty ml of a solution containing 1% (W/W) of salmon DNA was introduced into the medicament chamber of an ultrasonic nebulizer. Addition of a liposome suspension (125 μmole as phospholipid) thereto gave a pharmaceutical formulation for a human or animal.

## Example 25

Twenty ml of a solution containing 86 μmole of trypsin was introduced into the medicament chamber of an ultrasonic nebulizer. Addition of a liposome suspension (125 μmole as phospholipid) thereto gave a pharmaceutical formulation for a human or animal.

## Experiment 1

After introduction of the pharmaceutical formulation obtained in Example 1, an ultrasonic nebulizer was operated for 30 minutes. The amount of IFN-γ remaining in the medicament chamber after 30 minutes was determined by ELISA (Biosource).

## Experiment 2

After introduction of the pharmaceutical formulation obtained in Example 2, a jet nebulizer was operated for 30 minutes. The amount of IFN-γ remaining in the medicament chamber after 30 minutes was determined by ELISA (Biosource).

## Experiment 3

After introduction of the pharmaceutical formulation obtained in Example 2, a jet nebulizer was operated for 30 minutes. Sprayed drops were captured in 8 ml of a physiological phosphate saline, and the amount of IFN-γ in the capturing solution was measured by ELISA (Biosource).

## Experiment 4

After introduction of the pharmaceutical formulation obtained in Example 6, a jet nebulizer was operated for 30 minutes. Sprayed drops were captured in 8 ml of a phosphate buffer, and the amount of IL-2 in the capturing buffer was measured by ELISA (Biosource).

## Experiment 5

After introduction of the pharmaceutical formulation obtained in Example 24, an ultrasonic nebulizer was operated for 30 minutes. Viscosity of the medicament chamber was measured by E-type viscosimeter. The residual amount of sDNA was calculated from the relation formula between the viscosity and sDNA concentration.

## Experiment 6

After introduction of the pharmaceutical formulation obtained in Example 25, an ultrasonic nebulizer was operated for 30 minutes. Activity of trypsin in the medicament chamber was determined from the rate of change in absorbance (253nm) at pH 8.0 and at 0.25°C using ethyl N-alpha-benzoyl-L-argininate as a substrate.

The results of Experiments 1-6 are shown in the following Table 1.

Table 1

| | | Residual Rate (%) | | | | | | Spraying Rate (%) | |
|---|---|---|---|---|---|---|---|---|---|
| Nebulizer | | Ultrasonic Nebulizer | | Jet Nebulizer | | | | | |
| Physiologically active substance | Trypsin | sDNA | IFN-γ | | | | | | IL-2 |
| Liposome free | 17 | 79.0 | 1.5 | 4.3 | 2.3 | 3.3 | 0.3 | 0.3 | 2.0 |

Table 1 (continued)

| Nebulizer | Residual Rate (%) | | | | | | Spraying Rate (%) | | |
|---|---|---|---|---|---|---|---|---|---|
| | Ultrasonic Nebulizer | | | | Jet Nebulizer | | | | |
| HSPC/DSPG = 10/1 | 33 | | 35.7 | | 24.1 | 32.6 | 15.3 | 39.7 | 10.7 |
| DSPC/DPPG = 10/1 | | | | | 31.6 | | 31.3 | | |
| EggPC/DSPG = 10/1 | | | | | 27.1 | 19.6 | 16.3 | 13.0 | |
| EggPC | | | | | 4.4 | 3.0 | 1.5 | 0.9 | |
| DPPC/CH/SA= 52/40/8 | | 102.3 | | | | | | | |

## Discussion based on the experimental results (As for interferon)

The experimental results show that when a pharmaceutical formulation was prepared by introducing an interferon γ solution together with a liposome suspension into the medicament chamber of a nebulizer, the amount of interferon γ remaining in the chamber after 30 minutes operation of the nebulizer was about 12 times and about 8 - 11 times larger than the amount remaining after operation without the liposome suspension respectively in the case of an ultrasonic nebulizer and a jet nebulizer (provided that 10EggPC is excluded in the latter). Table 1 indicates the residual ratio of a medicament with respect to the total amount initially charged. Since the amount of the medicament decreases up to one third (residual ratio: about 33%) after 30 minutes operation of a nebulizer, it is concluded that substantially more than 70% of interferon γ remained stable without decomposition due to the addition of liposome, except for EggPC.

Table 1 further shows that the amount of interferon γ after spraying in the case of the addition of liposome was about 49 - 104 times larger than the amount when liposome free, except for EggPC. This means that the addition of liposome kept interferon γ stable without decomposition.

It is understood that the stability of interferon γ may be changed depending on the kind of liposome (HSPC/DSPG = 10/1, DSPC/DPPG =10/1, EggPC/DSPC = 10/1, EggPC). Although only interferon γ was tested in this respect, it is believed that there exists an optimum combination of a particular medicament and the kind of liposome for the purpose of establishing high stability.

## (As for trypsin, sDNA, and IL-2)

Addition of liposome increased the stability of trypsin, sDNA, and IL-2 about 2 times, 1.3 times, and 5 times respectively, compared with liposome free.

## Industrial Applicability

When preparing a pharmaceutical formulation to be administered via a mucous membrane, especially a formulation to be inhaled by means of a jet nebulizer, an ultrasonic nebulizer, a metered dose inhaler, or a dry powder inhaler, high stability of a biologically active substance to be administered by the medicament administration device can be established by adopting a process of contacting the substance with liposomes or microspheres in an aqueous medium.

In another aspect of the present invention, when preparing a pharmaceutical formulation to be inhaled by utilizing a jet nebulizer, an ultrasonic nebulizer, a metered dose inhaler, or a dry powder inhaler in practice, for instance, dispensing in a hospital, the increase in stability of a biologically active substance can be attained by adopting a process of contacting the substance with a liposome or the like in an aqueous medium. Further, in another aspect of the invention, the above-mentioned biologically active substance and liposome and the like which are separately obtained and contacted can be used as a pharmaceutical formulation to be administered via a mucous membrane, which contains said substance.

## Claims

1.  A kit for preparing a pharmaceutical formulation for a human or an animal, which comprises a preparation of a biologically active substance and a preparation containing liposomes or microspheres, which preparations are used for production of the pharmaceutical formulation to be administered by a medicament administration device that disperses the biologically active substance in the air, said production being conducted by contacting the biologically active substance with the liposomes or microspheres in an aqueous medium.

2. The kit of Claim 1 wherein the medicament administration device that disperses the biologically active substance in the air is a jet nebulizer, an ultrasonic nebulizer, a metered dose inhaler, or a dry powder inhaler.

3. The kit of Claim 1 wherein the pharmaceutical formulation for a human or an animal is to be administered via a mucous membrane.

4. The kit of Claim 1 wherein the biologically active substance is one which is liable to lose stability when dispersed in the air.

5. The kit of Claim 1 wherein the biologically active substance is a peptide, a protein, or a nucleic acid.

6. The kit of Claim 5 wherein the peptide is a cytokine or a bone-related peptide.

7. The kit of Claim 6 wherein the cytokine is interferon.

8. The kit of Claim 5 wherein the protein is a protease, antibiotic protein, or anti-tumor protein.

9. The kit of Claim 5 wherein the nucleic acid is DNA or RNA.

10. The kit of Claim 9 wherein the DNA or RNA is an antisense.

11. The kit of any one of Claims 1 - 10 wherein the biologically active substance is interferon γ, and the kit is used for curative or prophylactic treatment for virus infections or pulmonary diseases.

12. The kit of any one of Claims 1 - 10 wherein the preparation of the biologically active substance and/or the preparation containing liposomes or microspheres is or are a formulation or formulations dispersed in an aqueous medium.

13. The kit of any one of Claims 1 - 10, which further comprises a container containing an aqueous medium.

14. The kit of any one of Claims 1 - 10 wherein the preparation of the biologically active substance and/or the preparation containing liposomes or microspheres is or are a lyophilized preparation.

15. A method for production of a pharmaceutical formulation for a human or animal to be administered by a medicament administration device that disperses a biologically active substance in the air, which comprises a step of contacting the biologically active substance with liposomes or microspheres in an aqueous medium.

16. A method for stabilizing a biologically active substance by contacting the biologically active substance with liposomes or microspheres, in the process for the production of a pharmaceutical formulation for a human or animal to be administered by a medicament administration device that disperses the biologically active substance in the air.

17. The method of Claim 15 or 16 wherein the medicament administration device is a jet nebulizer, an ultrasonic nebulizer, a metered dose inhaler, or a dry powder inhaler.

18. A pharmaceutical formulation for a human or animal to be administered by a medicament administration device that disperses a biologically active substance in the air, which was produced by contacting the biologically active substance with liposomes or microspheres in an aqueous medium.

19. The formulation of Claim 18 wherein the medicament administration device is a jet nebulizer, an ultrasonic nebulizer, a metered dose inhaler, or a dry powder inhaler.

20. A method for curative or prophylactic treatment of diseases of a human or animal, which comprises administering to the human or animal a pharmaceutical formulation for a human or animal to be administered by a medicament administration device that disperses a biologically active substance in the air, which was produced via a step of contacting the biologically active substance with liposomes or microspheres in an aqueous medium.

21. Use of liposomes or microspheres for the production of a pharmaceutical formulation for a human or animal to be administered by a medicament administration device that disperses a biologically active substance in the air, which is produced via a step of contacting the biologically active substance with liposomes or microspheres in an aqueous medium.

22. A metered dose inhaler or a dry powder inhaler, characterized in that it contains a pharmaceutical formulation for a human or animal which has been produced via a step of contacting the biologically active substance with liposomes or microspheres in an aqueous medium.

# INTERNATIONAL SEARCH REPORT

| | International application No. |
|---|---|
| | PCT/JP98/02374 |

**A. CLASSIFICATION OF SUBJECT MATTER**
Int.Cl⁶ A61K9/127, 9/107, 9/14, A61M13/00, 15/00

According to International Patent Classification (IPC) or to both national classification and IPC

**B. FIELDS SEARCHED**

Minimum documentation searched (classification system followed by classification symbols)
Int.Cl⁶ A61K9/127, 9/107, 9/14, A61M13/00, 15/00

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 8-99897, A (Shionogi & Co., Ltd.), April 16, 1996 (16. 04. 96) (Family: none) | 1-19, 21, 22 |
| A | JP, 4-210925, A (Duphar Internationale Reself B.V.), August 3, 1992 (03. 08. 92) & EP, 440289, A | 1-19, 21, 22 |
| A | JP, 3-34920, A (Otsuka Pharmaceutical Co., Ltd.), February 14, 1991 (14. 02. 91) & EP, 393707, A | 1-19, 21, 22 |
| A | JP, 63-211223, A (Clayton Foundation for Research), September 2, 1988 (02. 09. 88) & EP, 267050, A | 1-19, 21, 22 |
| A | JP, 61-200849, A (Sterling Drug Inc.), September 5, 1986 (05. 09. 86) & EP, 190926, A & GB, 2170815, A | 1-19, 21, 22 |

| ☒ | Further documents are listed in the continuation of Box C. | ☐ | See patent family annex. |
|---|---|---|---|

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| August 11, 1998 (11. 08. 98) | August 18, 1998 (18. 08. 98) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

| | International application No. |
|---|---|
| | PCT/JP98/02374 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP, 60-89414, A  (Sterling Drug Inc.), May 20, 1985 (20. 05. 85) & DE, 3430385, A  &  GB, 2145107, A & FR, 2550706, A | 1-19, 21, 22 |

Form PCT/ISA/210 (continuation of second sheet) (July 1992)

## INTERNATIONAL SEARCH REPORT

| International application No. |
|---|
| PCT/JP98/02374 |

**Box I    Observations where certain claims were found unsearchable (Continuation of item 1 of first sheet)**

This international search report has not been established in respect of certain claims under Article 17(2)(a) for the following reasons:

1. ☒ Claims Nos.: 20

   because they relate to subject matter not required to be searched by this Authority, namely:
   The subject matter of claim 20 relates to methods for treatment of the human body by therapy.

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

   ☐    No protest accompanied the payment of additional search fees.

Form PCT/ISA/210 (continuation of first sheet (1)) (July 1992)